# EUROPEAN PATENT APPLICATION

(11) **EP 1 387 168 A2**
(43) Date of publication of application: **04.02.2004**
(21) Application number: 03017110.2
(22) Date of filing: 28.07.2003
(51) Int. Cl.: G01N 33/53, C08F 220/00, G01N 33/543

(54) **Physiologically active substance-measuring reagent and method for measuring physiologically active substance**

(30) Priority: 29.07.2002 JP 2002220110
(71) Applicant: JSR Corporation, Tokyo (JP)
(72) Inventor: Kasai, Kiyoshi, JSR Corporation, Tokyo (JP); Masukawa, Tohru, JSR Corporation, Tokyo (JP)
(74) Representative: Leson, Thomas Johannes Alois, Dipl.-Ing.

(57) **Abstract**

A physiologically active substance-measuring reagent and a method for measuring a substance to be measured using the reagent. The physiologically active substance-measuring reagent comprises particles of a support polymer obtained by radical emulsion polymerization of the components (1), (2) and (3) as described herein above, and a physiologically active substance having an interaction with a substance to be measured, supported on the particles.

## Description

### FIELD OF THE INVENTION

The present invention relates to support polymer particles obtained by emulsion polymerization of monomers having a specific composition. More particularly, it relates to a physiologically active substance-measuring agent wherein the particle surface is sensitized by a covalent binding method with a protein substance such as an antibody, antigen, enzyme, or hormone, a nucleic acid substance such as DNA or RNA, or a physiologically active sugar chain compound (hereinafter these compounds are referred to as "physiologically active substances"), and a method for measuring a physiologically active substance using the reagent.

### DESCRIPTION OF THE RELATED ART

Hitherto, as support polymer particles for use in the medical and biological fields, there has been widely used mainly polystyrene particles for physical adsorption sensitization and mainly carboxyl group-modified polystyrene particles for covalent binding sensitization.

Of those, polystyrene particles have a characteristic feature that they have a high physical adsorption capacity with proteins and are capable of easily adsorbing (sensitizing) an objective physiologically active substance, so that they have been used for a wide range of physiologically active substances. However, since the polystyrene-based particles result in adsorption of a large amount of non-objective other physiologically active substances present in a test analyte (this adsorption is referred to as non-specific adsorption), the non-specific adsorption inhibits the performance of the sensitized particles and is a severe problem in their use. As a countermeasure, a method of blocking is used wherein after the particle surface is sensitized with an objective physiologically active substance, the remaining particle surface is adsorbed with a less harmful protein such as bovine serum albumin (BSA), but the effect is not complete. It has been known to improve the performance as support particles for a physiologically active substance by copolymerizing polystyrene particles with a styrenesulfonic acid salt or an acrylic ester having a specific oligoethylene oxide side chain or oligopropylene oxide side chain or by hydrolyzing a fragment of a persulfate-based initiator bound to the particles through a heat-treatment in an alkaline aqueous solution after emulsion polymerization of the particles, but the effect is also insufficient. Moreover, the sensitization by the physical adsorption involves an essential problem that part of the sensitized physiologically active substance is sometimes desorbed during the use or storage of the sensitized particles.

On the other hand, in the covalent binding sensitization using polymer particles having an active functional group such as a carboxyl group, an amino group or a glycidyl group capable of covalently binding to a physiologically active substance, there are advantages that the physiologically active substance is hardly detached owing to the strong binding onto the particle surface, only an objective physiologically active substance can be allowed to exist for sensitization at the covalent binding sensitization, and the like. Therefore, the sensitized particles obtained by the covalent binding sensitization are generally excellent in performance stability but have limitations on physiologically active substances capable of covalently binding to the particles and thus limitations of a narrow range of applicable targets. Moreover, the surface of particles for the covalent binding sensitization is highly hydrophilic due to the existence of an active functional group such as a carboxyl group, an amino group, or a glycidyl group, so that affinity to physiologically active substances is low as compared with hydrophobic polystyrene particles for physical adsorption. As a result, the covalent binding method involves problems of a poor sensitization efficiency, a low performance such as sensitivity, a narrow range of performance control, a necessity of a large amount of an expensive sensitizing antibody, and a high production cost.

To overcome those problems of the covalent binding sensitization, there are a proposal wherein a compromise between the physical adsorption method and the covalent binding method is aimed by using a very minute amount of a carboxyl group on the particle surface, a proposal of particles having a hydroxyl group or an acrylic ester structure of a side chain of a specific structure on the particle surface, a proposal of particles having a large number of epoxy groups on the particle surface, a proposal of particles having a primary amine on the particle surface, a proposal using carboxyl group-modified particles having an increased carboxylic acid content on the particle surface by polymerizing first a monomer mixture having a high carboxylic acid monomer concentration at an early stage of the polymerization, and the like, but they are all insufficient.

### SUMMARY OF THE INVENTION

Accordingly, an object of the present invention is to use support particles excellent in storage stability of reagent particles, sensitivity, and linearity of sensitivity as covalent binding support particles for diagnostics.

The present invention provides a physiologically active substance-measuring reagent comprising particles of a support polymer obtained by radical emulsion polymerization of:
(1) 0.1 to 20% by weight of a radically polymerizable vinyl monomer having a carboxylic group,
(2) 0.05 to 20% by weight of at least one of a compound represented by the following formula (I):

   CH₂=C(R¹)CO(OCH₂CH₂)ₙOR² (I)

   wherein R¹ represents a hydrogen atom or a methyl group, R² represents a hydrogen atom, a C₁ to C₆ alkyl group, an alkoxyphenyl group, a phenyl group, an acryloyl group, or a methacryloyl group, and n represents a number of 2 to 22, and a radically polymerizable vinyl monomer having a strong acid group, and
(3) 60 to 99.8% by weight of a radically polymerizable vinyl monomer copolymerizable with the monomers (1) and (2), and
   a physiologically active substance having an interaction with a substance to be measured, supported on the particles.

The present invention further provides a method for measuring a substance to be measured using the reagent.

### DETAILED DESCRIPTION OF THE INVENTION

The present invention is described in detail below.

The radically polymerizable vinyl monomer having a carboxylic group that can be used in the present invention is a polymerizable unsaturated carboxylic acid, and examples thereof include acrylic acid, methacrylic acid, propionic acid, itaconic acid, fumaric acid, maleic acid, maleic anhydride, 2-carboxyethyl acrylate and a 2-carboxyethyl acrylate oligomer. Of those, acrylic acid, methacrylic acid, propionic acid, itaconic acid or fumaric acid is preferably used. Those acids can be used alone or in combination thereof.

The amount of the radically polymerizable vinyl monomer having a carboxylic group used is 0.1 to 20% by weight, preferably 0.5 to 10% by weight, more preferably 1 to 6% by weight, based on the total weight of the monomers used. When the amount is less than 0.1% by weight, the covalent binding sensitization is difficult to carry out due to the insufficient reacting group for covalent binding to a physiologically active substance. When the amount exceeds 20% by weight, stability in the emulsion polymerization becomes poor due to too high hydrophilicity of the monomers, and hence, a large amount of polymerization aggregates generates.

The radically polymerizable vinyl monomer having a strong acid group that can be used in the present invention is a monomer having a sulfonic acid group or a sulfuric acid group. Examples of the monomer include styrenesulfonic acid, 2-sulfoethyl methacrylate, 2-acrylamido-2-methylpropanesulfonic acid, 1-allyoxy-2-hydroxypropanesulfonate, and sodium, potassium and ammonium salts thereof. Those monomers can be used alone or in combination thereof. Of those, styrenesulfonic acid and salts thereof are preferably used from the point that those exhibit a large effect to decrease the non-specific adsorption of particles.

The amount of the radically polymerizable vinyl monomer having a strong acid group used is 0.05 to 20% by weight, preferably 0.1 to 10% by weight, more preferably 0.5 to 6% by weight, based on the total weight of the monomers used. When the amount is less than 0.05% by weight, a substantial effect cannot be obtained. When the amount exceeds 20% by weight, stability in the emulsion polymerization becomes poor due to too high hydrophilicity of the monomers, and hence, a large amount of polymerization aggregates generates.

The monomer represented by the above formula (I) has a well balanced hydrophilicity and hydrophobicity of the particle surface depending on the length of the polyethylene glycol structure, so that the strength of a steric hindrance protecting barrier of the particle surface can be adjusted.

In the formula (I), examples of the C₁ to C₆ alkyl group include methyl, ethyl and propyl, examples of the alkoxyphenyl group include methoxyphenyl, ethoxyphenyl and propoxyphenyl, and n is 2 to 22, preferably 3 to 14.

The amount of the compound represented by the formula (I) used is 0.05 to 20% by weight, preferably 0.1 to 10% by weight, more preferably 0.2 to 6% by weight, based on the total weight of the monomers used. When the amount is less than 0.05% by weight, a substantial effect cannot be obtained. When the amount exceeds 20% by weight, stability in the emulsion polymerization becomes poor due to too high hydrophilicity of the monomers, and hence, a large amount of polymerization aggregates generates.

Where the radically polymerizable vinyl monomer having a strong acid group and the compound represented by the formula (I) are used in combination, those are used such that the total amount of both compounds is 0.05 to 20% by weight based on the total weight of the monomers used.

Examples of the radically polymerizable vinyl monomer copolymerizable with those monomers include aromatic vinyl compounds such as styrene, vinyltoluene, α-methylstyrene, divinylbenzene or vinylnaphthalene; acrylates or methacrylates, such as methyl acrylate, methyl methacrylate, butyl methacrylate, 2-ethylhexyl acrylate, t-butyl methacrylate or cyclohexyl methacrylate; vinyl ester compounds such as vinyl acetate, vinyl formate or allyl acetate; polymerizable double bond-containing cyan compounds such as acrylonitrile, methacrylonitrile or vinylidene cyanide; and other polymerizable double bond-containing compounds such as vinyl chloride, vinylidene chloride, vinyl methyl ketone, vinyl methyl ether or vinyl ethyl ether. Of those, styrene is particularly preferred from the standpoint of the compatibility with a physiologically active substance. Those monomers can be used alone or in combination thereof.

The amount of the radically polymerizable vinyl monomer copolymerizable with the monomers (1) and (2) used is 60 to 99.8% by weight.

The emulsion polymerization used in the present invention can employ the conventional emulsion polymerization. A polymerization initiator used in the emulsion polymerization is a water-soluble radical initiator, and examples thereof include sodium persulfate, potassium persulfate, ammonium persulfate, hydrogen peroxide/ferrous sulfate, cumene hydroperoxide/ascorbic acid, 2,2'-azobisisobutyronitrile and 2,2'-azobis-2,4-dimethylvaleronitrile. Of those, persulfates are preferably used.

The emulsifier used in the present invention can be the conventional emulsifiers. Specifically, anionic surfactants such as dodecylbenzenesulfonates or dodecyl sulfates are preferably used. Depending on the applications, it is possible to use a nonionic emulsifier or to use a nonionic emulsifier in combination with an anionic emulsifier. Further, it is also possible to use an anionic/nonionic emulsifier having an anionic group and a nonionic group in one molecule.

The emulsion polymerization can be carried out without using an emulsifier, and a so-called soap-free polymerization that does not use an emulsifier is also encompassed in the definition of the emulsion polymerization according to the present invention.

There are limitations in the soap-free polymerization to control the particle size, but the polymerization has an advantage that the particles polymerized by this method does not contain adsorbed emulsifier on the surface thereof, and hence the adsorption of a physiologically active substance is satisfactory.

The particle size of the polymer particles for physiologically active substance support is 0.03 to 2 µm, preferably 0.05 to 1 µm, more preferably 0.07 to 0.8 µm.

The polymer particles for physiologically active substance support according to the present invention can be used as support particles for use in medical and biological fields by adjusting pH after the emulsion polymerization, removing remaining monomers, if necessary, and washing the particle surface through dialysis, ultrafiltration, centrifugation or the like.

The polymer particles for physiologically active substance support have a carboxylic acid group on the particle surface and can bind to a physiologically active substance by the covalent binding method. The particles result in less non-specific adsorption of physiologically active substances as compared with the conventional carboxylic acid-modified particles, and thus can be utilized as high performance immunodiagnostics, biological support particles, nucleic acid-trapping particles, and the like.

The polymer particles for physiologically active substance support of the present invention can be used after binding a physiologically active substance such as a protein, nucleic acid, or sugar chain substance directly to the particle surface by the covalent binding method. Alternatively, the physiologically active substance can be spaced at a slight distance from the particle surface by binding one end of a bifunctional spacer compound onto the particle surface and another end to the physiologically active substance. For example, ethylene glycol diglycidyl ether and derivatives thereof can be employed as the spacer compounds. By spacing the physiologically active substance from the particle surface, the activity of the physiologically active substance is considerably improved to reach close to the activity value in a homogeneous solution, and sometimes it is expectable to exceed the value.

The sensitization of the support particles of the invention with a physiologically active substance such as an antigen, antibody, protein, streptoavidin, nucleic acid, or sugar chain substance is carried out by the covalent binding method. As the sensitization method, a conventional usual process (protocol) of the covalent binding method is applicable.

The antigen or antibody in the invention is not particularly limited as far as it reacts with a component generally contained in an analyte. Examples thereof include antigens or antibodies for coagulation fibrinolysis-related assay such as an anti-antiplasmin antibody for antiplasmin assay, anti-D dimer antibody for D dimer assay, anti-FDP antibody for FDP assay, anti-tPA antibody for tPA assay, antithrombin=antithrombin complex antibody for TAT assay, and FPA antibody for FPA assay; antigens or antibodies for tumor-related assay such as an anti-BFP antibody for BFP assay, anti-CEA antibody for CEA assay, anti-AFP antibody for AFP assay, antiferritin antibody for ferritin assay, and anti-CA19-9 antibody for CA19-9 assay; antigens or antibodies for serum protein-related assay such as an antiapolipoprotein antibody for apolipoprotein assay, anti-β2-microgrobulin antibody for β2-microgrobulin assay, anti-α1-microgrobulin antibody for α1-microgrobulin assay, antiimmunogrobulin antobody for immunogrobulin assay, and anti-CRP antibody for CRP assay; antigens or antibodies for endocrine function-related assay such as anti-HCG antibody for HCG assay; antigens or antibodies for infectious disease-related assay such as anti-HBs antibody for HBs antigen assay, HBs antigen for HBs antibody assay, HCV antigen for HCV antibody assay, HIV-1 antigen for HIV-1 antibody assay, HIV-2 antigen for HIV-2 antibody assay, HTLV-1 antigen for HTLV-1 assay, mycoplasma antigen for mycoplasmosis assay, toxoplasma antigen for toxoplasmosis assay, and storeptolysin O antigen for ASO assay; antigens or antibodies for autoimmune-related assay such as DNA antigen for anti-DNA antibody assay and thermally denatured human IgG for RF assay; antigens or antibodies for drug analysis such as antilidocaine antibody for lidocaine assay, and the like, but are not limited thereto. The antibody used may be a polyclonal antibody or a monoclonal antibody.

The method for sensitizing the support particles of the invention with a physiologically active substance includes the following method but is not particularly limited thereto. Sensitized particles immobilizing a physiologically active substance on the support particles may be obtained by dispersing the support particles in water or a buffer solution, adding a coupling agent such as an N-hydroxyimide or a water-soluble carbodiimide including 1-ethyl-3-(3-dimethylaminopropyl)carbodiimide (EDC), stirring the resulting mixture at 20 to 50°C for 0 to 2 hours, adding a physiologically active substance, and finally stirring at 20 to 50°C for 1 to 16 hours. At that time, pH of the reaction mixture is preferably 4.5 to 8.5.

The amount of the water-soluble carbodiimide and N-hydroxyimide used is preferably 5 to 500 mg per 1 g of the support particles.

The amount of the physiologically active substance added is preferably 5 to 80 mg per 1 g of the support particles.

An example of measuring a physiologically active substance according to the invention is that the support particles are sensitized with an antibody, and an antigen substance having an immunologic activity against the antibody can be measured. Another example is that when sensitized particles to which a biotin marker, storeptoavidin, is bound and a substance having an immunologic activity to the biotin marker are mixed, particles are bound to each other through an immunological reaction, and aggregation occurs. The measurement of the physiologically active substance can be carried out by determining quantitatively the degree of aggregation by an optical measurement and measuring the immunologically active substance to be measured.

The present invention is described in more detail by reference to the following examples. It should however be understood that the invention is not construed as being limited thereto.

In the Examples, all percents and parts are by weight unless otherwise indicated.

### EXAMPLE 1

700 parts of water, 0.5 part of sodium dodecylbenznesulfonate, 0.05 part of sodium carbonate, 95 parts of styrene, 2 parts of acrylic acid and 1 part of sodium styrenesulfonate were placed in a 1 liter four-neck flask equipped with a stirrer, the atmosphere in the flask was replaced with nitrogen gas, and the flask was heated to 80°C. When the temperature reached 80°C, 10 parts of 5% ammonium persulfate was added the flask to initiate polymerization. After the polymerization was continued at 80°C for 6 hours, the flask was cooled and the contents were filtered through a 500 mesh wire net filter. The amount of the resulting solid product was measured to calculate a polymerization conversion. As a result, it was found to be 99.7%. The amount of aggregates was 0.01% or less and thus a good polymerization stability was exhibited. The particle size was measured by a dynamic light scattering method. As a result, it was found to be 125 nm.

The particles were purified by repeating three times the operations of adjusting pH to 8 with 1% sodium hydroxide, centrifuging the particles with 10,000 rpm × 30 minutes to precipitate the particles, removing the resulting supernatant, and re-dispersing the solid product into ion-exchanged water.

### EXAMPLES 2 TO 9 AND COMPARATIVE EXAMPLES 1 TO 6

Particles of Examples 2 to 9 and Comparative Examples 1 to 6 were synthesized in the same manner as in Example 1, except that the kind and amount of the monomers used were changed as shown in Tables 1 and 3 below.

The results obtained are shown in Tables 2 and 4 below.

**TABLE 1**

| | Example | | | | | | | | |
|---|---|---|---|---|---|---|---|---|---|
| Monomer Composition | 1 | 2 | 3 | 4 | 5 | 6 | 7 | 8 | 9 |
| Styrene | 97 | 96 | 94 | 85 | 94 | 79 | 92 | 94 | 94 |
| Acrylic acid | 2 | 1 | - | - | - | - | 1 | - | - |
| Methacrylic acid | - | - | 5 | 10 | 5 | 5 | - | 5 | 5 |
| Itaconic acid | - | 2 | - | - | - | - | 2 | - | - |
| Fumaric acid | - | - | - | 1 | - | - | - | - | - |
| Sodium styrnesulfonate | 1 | 1 | 1 | 5 | - | 1 | - | - | - |
| 2-Acrylamido-2-methylpropanesulfonic acid | - | - | - | - | 1 | - | - | - | - |
| Methoxy(oligoethylene glycol n=2) methacrylate | - | - | - | - | - | 15 | - | - | - |
| Methoxy(oligoethylene glycol n=4) methacrylate | - | - | - | - | - | - | 5 | - | - |
| Methoxy(oligoethylene glycol n=9) methacrylate | - | - | - | - | - | - | - | 1 | - |
| Methoxy(oligoethylene glycol n=22) methacrylate | - | - | - | - | - | - | - | - | 1 |

**TABLE 2**

| Polymerization Results | Example | | | | | | | | |
|---|---|---|---|---|---|---|---|---|---|
| | 1 | 2 | 3 | 4 | 5 | 6 | 7 | 8 | 9 |
| Polymerization conversion (%) | 99.7 | 99.2 | 99.9 | 99 | 99.2 | 99.8 | 99.8 | 99.4 | 99 |
| Polymerization stability | ⓞ | ⓞ | ⓞ | ○ | ○ | ○ | ○ | ○ | ○ |
| Particle size (nm) | 125 | 110 | 115 | 135 | 102 | 372 | 124 | 135 | 115 |
| Evaluation Results of Sensitized Particles | | | | | | | | | |
| Dilution factor of antiserum | 2560 0 | 2560 0 | 1280 0 | 6400 | 1280 0 | 6400 | 1280 0 | 6400 | 3200 |
| Sensitivity | ⓞ | ⓞ | ⓞ | ○ | ⓞ | ○ | ⓞ | ○ | ○ |
| Notes: | | | | | | | | | |
| ⓞ Excellent | | | | | | | | | |
| O: Good | | | | | | | | | |

**TABLE 3**

| | Comparative Example | | | | | |
|---|---|---|---|---|---|---|
| Monomer Composition | 1 | 2 | 3 | 4 | 5 | 6 |
| Styrene | 100 | 98 | 98 | 98 | 73 | 70 |
| Acrylic acid | - | 2 | - | - | 2 | - |
| Methacrylic acid | - | - | - | - | - | 5 |
| Itaconic acid | - | - | - - | - | - | - |
| Fumaric acid | - | - | - | - | - | - |
| Sodium styrnesulfonate | - | - | 2 | - | 25 | - |
| 2-Acrylamido-2-methylpropanesulfonic acid | - | - | - | - | - | - |
| Methoxy(oligoethylene glycol n=2) methacrylate | - | - | - | - | - | - |
| Methoxy(oligoethylene glycol n=4) methacrylate | - | - | - | 2 | - | 25 |
| Methoxy(oligoethylene glycol n=9) methacrylate | - | - | - | - | - | - |
| Methoxy(oligoethylene glycol n=22) methacrylate | - | - | - | - | - | - |

**TABLE 4**

| Polymerization Results | Comparative Example | | | | | |
|---|---|---|---|---|---|---|
| | 1 | 2 | 3 | 4 | 5 | 6 |
| Polymerization conversion (%) | 99.8 | 99.6 | 99.2 | 99.1 | 78 | 67 |
| Polymerization stability | ○ | ○ | ○ | ○ | × | × |
| Particle size (nm) | 120 | 105 | 135 | 121 | 150 | 168 |
| Evaluation Result of Sensitized Particles | | | | | | |
| Dilution factor of antiserum | 100 | 400 | 100 | 100 | 800 | 800 |
| Sensitivity | × | × | × | × | Δ | Δ |
| Notes: | | | | | | |
| O: Good | | | | | | |
| Δ: Slightly poor | | | | | | |
| × : Poor | | | | | | |

### Preparation of reagent from latex

1 ml of 10 mg/ml of 1-ethyl-3-(3-dimethylaminopropyl)carbodiimide (EDC) and 10 ml of an hGC antibody solution obtained by adding an hCG antibody to MES solution (pH 6.1) so as to be a concentration of 1 mg/ml were added to 10 ml of a 1% by weight suspension of the purified particles suspended in MES solution (pH 6.1), followed by stirring at room temperature for 3 hours. The resulting particles were washed by carrying out three times operations of centrifugation of 16,000 rpm x 30 minutes at 4°C, removal of the resulting supernatant, and re-dispersion into MES solution, and were suspended into a blocking buffer containing 1 % serum albumin to prepare a 0.1% suspension of hGC-sensitized particles. Evaluation of sensitized particles

With regard to the reactivity with anti-hGC antiserum, individual sensitized particles were compared. The anti-hGC antiserum was diluted by factors of 100, 200, 400, 800, 1600, 3200, 6400, 12800, 25600, and 51200 to prepare a dilution series of 10 steps. The sensitized particles were added to each dilution and a maximum dilution factor at which aggregation of the particles was observed was determined, whereby the particles were evaluated. The results are shown in Tables 2 and 4 above.

Latex diagnostic particles exhibiting a high sensitivity, a low background and a wide measurable range, specific nucleic acid-trapping particles capable of trapping, purifying and concentrating a specific DNA or RNA, specific protein-separating particles, particles for collecting a control factor protein of DNA transcription, and the like can be prepared by using the support particles according to the present invention. In addition, an affinity chromatography for detecting and collecting a specific physiologically active substance can be prepared by packing a column with those particles. Further, it is possible to use those particles with laying them on a plate.

A component having an affinity to the particles can be fractionated or separated by binding a medical drug candidate to the surface of the polymer particles for physiologically active substance support of the present invention, and introducing a cell decomposition product or protein to a column having packed with the particles, a filter or thin-layer gel having the particles supported thereon, or a plate having the particles spotted thereon. As a result, a biological site interacting with the medical drug candidate can be detected. Alternatively, a medical drug substance can be screened by binding a specific physiologically active substance, such as a protein, nucleic acid or sugar chain, to the surface of the polymer particles for physiologically active substance support of the present invention, introducing a medical drug candidate to a column having packed with the particles, a filter or thin-layer gel having the particles supported thereon, or a plate having the particles spotted thereon, and fractionating or separating a component having an affinity to the particles.

It should further be apparent to those skilled in the art that various changes in form and detail of the invention as shown and described above may be made. It is intended that such changes be included within the spirit and scope of the claims appended hereto.

This application is based on Japanese Patent Application No. 2002-220110 filed July 29, 2002, the disclosure of which is incorporated herein by reference in its entirety.

A physiologically active substance-measuring reagent and a method for measuring a substance to be measured using the reagent. The physiologically active substance-measuring reagent comprises particles of a support polymer obtained by radical emulsion polymerization of the components (1), (2) and (3) as described herein above, and a physiologically active substance having an interaction with a substance to be measured, supported on the particles.

## Claims

1. A physiologically active substance-measuring reagent comprising particles of a support polymer obtained by radical emulsion polymerization of:
(1) 0.1 to 20% by weight of a radically polymerizable vinyl monomer having a carboxylic group,
(2) 0.05 to 20% by weight of at least one of a compound represented by the following formula (I):
CH₂=C(R¹)CO(OCH₂CH₂)ₙOR² (I)
wherein R¹ represents a hydrogen atom or a methyl group, R² represents a hydrogen atom, a C₁ to C₆ alkyl group, an alkoxyphenyl group, a phenyl group, an acryloyl group, or a methacryloyl group, and n represents a number of 2 to 22, and a radically polymerizable vinyl monomer having a strong acid group, and
(3) 60 to 99.8% by weight of a radically polymerizable vinyl monomer copolymerizable with the monomers (1) and (2), and supported on the particles, a physiologically active substance having an interaction with a substance to be measured.

2. The physiologically active substance-measuring reagent as claimed in claim 1, wherein the radically polymerizable vinyl monomer having a strong acid group is styrenesulfonic acid or a styrenesulfonic acid salt.

3. A method for measuring a physiologically active substance which comprises measuring a substance to be measured by an interaction between the physiologically active substance supported on the physiologically active substance-measuring reagent as claimed in claim 1 and the substance to be measured in a sample.

4. The method for measuring a physiologically active substance as claimed in claim 3, wherein the interaction between the substance to be measured and the physiologically active substance supported on the physiologically active substance-measuring reagent is aggregation of the physiologically active substance-measuring agent.

5. The method for measuring a physiologically active substance as claimed in claim 3, wherein the interaction between the substance to be measured and the physiologically active substance supported on the physiologically active substance-measuring reagent is adsorption of the substance to be measured, with the physiologically active substance.
